# EUROPÄISCHE PATENTANMELDUNG

(11) **EP 1 136 565 A1**
(43) Veröffentlichungstag der Anmeldung: **26.09.2001**
(21) Anmeldenummer: 00106175.3
(22) Anmeldetag: 21.03.2000
(51) Int. Cl.: C12Q 1/68, C12N 15/10

(54) **Verfahren zur Charakterisierung membranassoziierter Polypeptide**

(71) Anmelder: Barth, Stefan, Dr.rer.nat.Dr.rer.medic., Medizinische Universitätsklinik I LFI, Ebene 4, R702, 50924 Köln (DE)
(72) Erfinder: Engert, Andreas, 50931 Köln (DE); Barth, Stefan, 50674 Köln (DE); Weidenmüller, Ute, 50354 Hürth (DE)
(74) Vertreter: Meyers, Hans-Wilhelm, Dr.Dipl.-Chem.

(57) **Zusammenfassung**

Verfahren zur Selektion membranassoziierter Polypeptide und komplementärer Bindungspeptide umfassend folgende Schritte:
- Erstellung einer ersten cDNA- Expressionsbibliothek für Bindungspeptide,
- Erstellung einer zweiten cDNA-Expressionsbibliothek einer Zelle,
- Immobilisierung einer der cDNA-Expressionsbibliotheken,
- Screening der immobilisierten cDNA-Expressionsbibliothek mit der anderen cDNA-Expressionsbibliothek,
wobei die erste cDNA-Expressionsbibliothek auf komplementäre Bindungspeptide angereichert und die zweite cDNA-Expressionsbibliothek eine subtraktive Bibliothek sein kann.

## Beschreibung

Die vorliegende Erfindung betrifft ein Verfahren zur Selektion und Charakterisierung membranassoziierter Polypeptide und komplementärer Bindungspeptide.

Zellen tragen an ihrer Außenseite, in die Membran eingebettete Proteine, die eine Vielzahl verschiedener Funktionen im Bereich von Transport, Kommunikation und Energieübertragung übernehmen. Die Identifizierung dieser membranassoziierten Proteine und ihrer Funktion ist von großem Interesse für die biochemische und pharmazeutische Forschung.

Übliche Wege zur Identifizierung solcher membranassoziierter Proteine gehen beispielsweise von spezifischen Antikörpern aus, die Strukturen auf der interessierenden Zelle - aber nicht auf anderen Zellen - erkennen. Die anschließende Isolierung der so detektierten Strukturen der Zelle erfolgt dann beispielsweise mittels aufwendiger chromatographischer Verfahren.

Die vorliegende Erfindung hat sich zur Aufgabe gestellt, ein Verfahren zur Selektion und Charakterisierung membranassoziierter Polypeptide und komplementärer Bindungspeptide bereitzustellen, durch das leichter und schneller die relevanten Informationen erhalten werden können.

Das erfindungsgemäße Verfahren zur Selektion membranassoziierter Polypeptide und komplementärer Bindungspeptide umfasst folgende Schritte:
a) Erstellung einer ersten cDNA-Expressionsbibliothek für komplementäre Bindungspeptide,
b) Erstellung einer zweiten cDNA-Expressionbibliothek einer Zelle,
c) Immobilisierung einer der cDNA-Expressionsbibliotheken,
d) Screening der immobilisierten cDNA-Expressionsbibliothek mit der anderen cDNA-Expressionsbibliothek,
wobei die erste cDNA-Expressionsbibliothek auf komplementäre Bindungspeptide nach beschriebenen Verfahren angereichert und die zweite cDNA-Expressionsbibliothek eine subtraktive Bibliothek sein kann.

"Membranassoziierte Polypeptide" sind insbesondere Proteine, die in oder auf der äußeren Zellmembran angelagert oder verbunden sind.

"Komplementäre Bindungspeptide" sind Polypeptide, die spezifisch mit den membranassoziierten Proteinen in Wechselwirkung treten. Eine spezifische Wikrung bedeutet eine Dissoziationskonstante K_{D}<10⁻⁴ mol/l, bevorzugt <10⁻⁶ mol/l.

Eine "cDNA-Expressionsbibliothek für Bindungspeptide" bezeichnet eine Vielzahl von Substanzen, bei denen in spezifischer Weise in einer Einzelkomponente die kodierende Region einer Nukleinsäure mit dem dazugehörenden exprimierten bindungsaktiven Polypeptid verknüpft ist. Typische Beispiele hierfür sind Bibliotheken komplementärer Bindungspeptide in transformierten Zellen (bevorzugt Bakterien und Hefen, besonders bevorzugt Bakteriophagen), bei denen die eingebaute Nukleinsäure funktionell translatiert wird und das exprimierte Protein insbesondere als Fusionsprotein an der Oberfläche der Zelle verankert ist, so dass ausgehend von den Eigenschaften des Bindungspeptids eine Analyse seiner Bindungsfunktion mit dem membranassoziierten Polypeptid nach derzeitigem Stand der Technik durchgeführt werden kann und gleichzeitig die damit korrelierende Nukleinsäure eindeutig zugeordnet werden kann.

Die Erstellung von cDNA-Expressionsbibliothek für komplementäre Bindungspeptide in Bakteriophagen ist beispielsweise beschrieben im US Patent 5,969,108.

Eine "cDNA-Expressionsbibliothek einer Zelle" bezeichnet eine Vielzahl von Substanzen, bei denen in spezifischer Weise in einer Einzelkomponente die zu einer cDNA umgewandelte kodierende Region einer mRNA mit dem dazugehörenden exprimierten Polypeptid verknüpft ist. Typisches Beispiel hierfür sind cDNA-Expressionsbibliotheken in λ-Phagen, die gewährleisten, dass die eingebauten Nukleinsäuren funktionell in Bakterien translatiert wird und nach Lyse der Bakterien das exprimierte Protein in entsprechenden Plaques exponiert ist. Diese cDNA-Expressionsbibliotheken beinhalten somit das Abbild der einer Zellen exprimierten Proteine einer Zelle, so dass ausgehend von den Eigenschaften membranassoziierter Polypeptide durch Kombination mit dem entsprechenden komplementären Bindungspeptid nach derzeitigem Stand der Technik die Interaktion über Farbreaktionen dokumentiert werden können.

cDNA-Expressionssysteme, die erfindungsgemäß eingesetzt werden können sind beispielsweise Uni ZAP in Gigapack III Gold Kit der Firma Stratagene oder TriplEx™ der Firma Clontech oder ähnliche pro- oder eukaryotische cDNA-Expressionssysteme.

"Immobilisierung" bedeutet, dass die Mitglieder der einen Bibliothek an einer festen Matrix gebunden werden, so dass sie besonders leicht von einer wässrigen Lösung getrennt werden können.

"Anreicherung der ersten cDNA-Expressionsbibliothek gegen membranassoziierte Polypeptide" bedeutet, dass die erste cDNA-Expressionsbibliothek mit den interessierenden Zellen, aus denen die zweite cDNA-Expressionsbibliothek erstellt worden ist, in Kontakt gebracht wird und - gegebenenfalls nach durchzuführenden Waschschritten - die Mitglieder der cDNA-Expressionsbibliothek angereichert werden, die mit einer hinreichenden Dissoziationskonstante an die Zellen binden und deswegen in der Bibliothek verbleiben (beschrieben im US Patent 5.969.108). Durch mehrere Bindungsrunden kann die Anreicherung soweit gehen, dass nur noch einzelne Mitglieder der Bibliothek erhalten bleiben, d.h. eine Vereinzelung eintritt. Alternativ hierzu kann auch eine Depletion der Bibliothek gegen membranassoziierte Polypeptide gegen Zellen die die entsprechenden membranassoziierten Polypeptide nicht aufweisen oder gegen nicht oberflächenspezifische Proteine von Zellen erfolgen.

Eine subtraktive Bibliothek bezieht sich auf die cDNA-Expressionbibliothek einer Zelle. Diese Bibliothek enthält vermehrt Polypeptide, die membranassoziiert vorliegen und an deren Analyse ein Interesse besteht. Derartige subtraktive Bibliotheken werden erstellt durch subtraktive Hybridisierung, d.h. die DNA eines Zelltyps wird mit der mRNA eines zweiten Zelltyps hybridisiert, die doppelsträngigen cDNA/mRNA-Hybride entfernt und nach Zerstörung der einzelstränigen freien mRNA die verbleibende cDNA kloniert. Durch Einsatz von einander ähnlichen oder verwandten Zellen (Tumorzelle, normale Ausgangszelle), von denen eine die relevanten membranassoziierten Polypeptide nicht aufweist, können so subtraktive Bibliotheken erstellt werden, die für die relevanten membranassoziierten Polypeptide stark angereichert sind. Verfahren zur Herstellung subtraktiver Bibliotheken sind beschrieben in Jin H. et al., Biotechniques 23(6), 1084-1086 (1997) oder Wu G et al., Genet Anal Tech Appl 11(2), 29-33 (1994) oder Lopez-Fernandez LA et del Mazo J, Biotechniques 14(4), 654-659 (1993).

"Screening der einen cDNA-Expressionsbibliothek an der anderen immobilisierten cDNA-Expressionsbibliothek" bedeutet, dass die eine cDNA-Expressionsbibliothek in wässriger Phase zu der immobilisierten gegeben wird, wobei membranassoziierte Polypeptide und komplementäre Bindungsproteine miteinander eine Wechselwirkung eingehen, so dass sich - gegebenenfalls nach durchzuführenden Waschschritten - in der immobilisierten cDNA-Expressionsbibliothek vermehrt Polypeptide nachweisen lassen, die eine hohe Affinität für die Polypeptide der anderen Bibliothek enthalten.

Geeignete komplementäre Bindungsproteine für die erste cDNA-Expressionsbibliothek sind Antikörper oder deren Derivate oder Fragmente, synthetische Peptide, Liganden, Lectine, Rezeptorbindungsmoleküle, Cytokine, Lymphokine, Chemokine, Adhäsionsmoleküle, die an Cluster of Differentiation (CD)-Antigene (CD2-CD154), sowie Cytokin-, Hormon-, Wachstumsfaktor-Rezeptoren oder Ionenpumpen oder kanalbildende Proteine und deren Mutanten binden.

Besonders interessante Zellen für die Erstellung der zweiten cDNA-Expressionsbibliothek sind Tumorzellen, da diese für immuntherapeutische oder -diagnostische Fragestellungen spezifische membranassoziierte Polypeptide aufweisen müssen.

Es ist sowohl möglich die erste cDNA-Expressionsbibliothek für Bindungspsptide an der Tumorzelle, die für die Erstellung der zweiten Bibliothek erstellt wird anzureichern, als auch die erste cDNA-Expressionsbibliothek gegen die normale nicht entartete Ausgangszelle, die das zu findende tumorassoziierte Oberflächenpeptid nicht aufweist zu depletieren.

Typischerweise kann die erste cDNA-Expressionsbibliothek nach üblichen Methoden aus B-Lymphozyten von nicht-immunisierten oder mit Zellen oder Zellfragementen humanen Spendern oder Säugern, insbesondere Mäusen oder Kaninchen generiert werden. Hierbei können in üblicher Weise die B-Lymphozyten aus lymphatischen Organen oder aus der Peripherie als Quelle für die DNA mit der Information für die Bindungspeptide verwendet werden.

Besonders geeignete cDNA-Expressionsbibliotheken werden in Bakteriophagen hergestellt, da diese einerseits mit einer sehr großen Diversität versehen werden können, andererseits sehr schnell vervielfacht und selektioniert werden können. Zur Konstruktion geeigneter cDNA-Expressionsbibliotheken werden sowohl filamentöse als auch λ-Phagen verwendet. Die Charakterisierung der isolierten membranassoziierten Polypeptide und ihrer komplementären Bindungspeptide erfolgt dann in üblicher Weise durch Sequenzierung und Datenbankanalyse.

## Patentansprüche

1. Verfahren zur Selektion membranassoziierter Polypeptide und komplementärer Bindungspeptide umfassend folgende Schritte:
• Erstellung einer ersten cDNA- Expressionsbibliothek für Bindungspeptide,
• Erstellung einer zweiten cDNA-Expressionsbibliothek einer Zelle,
• Immobilisierung einer der cDNA-Expressionsbibliotheken,
• Screening der immobilisierten cDNA-Expressionsbibliothek mit der anderen cDNA-Expressionsbibliothek,
wobei die erste cDNA-Expressionsbibliothek auf komplementäre Bindungspeptide angereichert und die zweite cDNA-Expressionsbibliothek eine subtraktive Bibliothek sein kann.

2. Verfahren nach Anspruch 1, **dadurch gekennzeichnet, dass** die erste cDNA-Expressionsbibliothek Antikörper oder deren Derivate oder Fragmente, synthetische Peptide, Liganden, Lectine, Rezeptorbindungsmoleküle, Cytokine, Lymphokine, Chemokine, Adhäsionsmoleküle, die an Cluster of Differentiation (CD)-Antigene (CD2-CD154), sowie Cytokin-, Hormon-, Wachstumsfaktor-Rezeptoren oder Ionenpumpen oder kanalbildende Proteine und deren Mutanten beinhaltet.

3. Verfahren nach einem der Ansprüche 1 oder 2, **dadurch gekennzeichnet, dass** die zweite cDNA-Expressionsbibliothek eine Expressionsbibliothek generiert aus der mRNA einer Tumorzelle ist.

4. Verfahren nach einem der Ansprüche 1 bis 3, dass die erste cDNA-Expressionsbibliothek an der Zelle, die für die Erstellung der zweiten cDNA-Expressionsbibliothek verwendet wird, angereichert wird.

5. Verfahren nach einem der Ansprüche 1 bis 4, **dadurch gekennzeichnet, dass** die erste cDNA- Expressionsbibliothek gegen eine ähnliche Zelle, die jedoch die membranassoziierten Oberflächenpeptide nicht aufweist, depletiert wird.

6. Verfahren nach einem der Ansprüche 1 bis 5, **dadurch gekennzeichnet, dass** die erste cDNA- Expressionsbibliothek ausgehend von B-Lymphozyten von nicht-immunisierten oder mit Zellen oder Zellfragementen von humanen Spendern oder Säugern, insbesondere Mäusen oder Kaninchen erstellt wird.

7. Verfahren nach einem der Ansprüche 1 bis 6, **dadurch gekennzeichnet, dass** die cDNA-Expressionsbibliotheken in eukaryotischen Zellen, Hefen, Bakterien oder Bakteriophagen angelegt sind.

8. Verfahren nach Anspruch 7, **dadurch gekennzeichnet, dass** die Phagen für die Bakteriophagenbibliotheken aus der Gruppe der filamenösen und λ-Phagen ausgewählt sind.

9. Verfahren nach einem der Ansprüche 1 bis 7, **dadurch gekennzeichnet, dass** eine Charakterisierung der membranassoziierten Polypeptide und komplementären Bindungspeptide zunächst durch Sequenzierung und Datenbankanalyse erfolgt.

10. Verwendung der membranassoziierten Polypeptide und deren komplementärer Bindungspeptide erhältlich durch ein Verfahren nach einem der Ansprüche 1 bis 9 zur Herstellung eines Arznei- oder Diagnostikmittels zur Diagnostik und Therapie von Tumorerkrankungen.
